# EUROPÄISCHE PATENTSCHRIFT

(11) **EP 1 374 824 B1**
(45) Veröffentlichungstag und Bekanntmachung des Hinweises auf die Patenterteilung: **18.01.2006**
(21) Anmeldenummer: 03013782.2
(22) Anmeldetag: 18.06.2003
(51) Int. Cl.: A61H 33/00, A01N 25/34, A61L 2/16, F16L 9/12

(54) **Teil eines warmwasserführenden Rohrsystems**
Part of a warm water containing pipe system
Pièce d'un système de conduite d'eau chaude

(30) Priorität: 21.06.2002 DE 20209632 U
(43) Veröffentlichungstag der Anmeldung: 02.01.2004
(73) Patentinhaber: Repabad GmbH, 73240 Wendlingen (DE)
(72) Erfinder: Stolz, Gunther, 73087 Boll (DE)
(74) Vertreter: Hoffmeister, Helmut

(56) Entgegenhaltungen:
- CH-A- 688 467
- DE-A- 4 447 189
- GB-A- 2 353 283
- US-A- 5 554 373
- US-A- 5 919 554
- PATENT ABSTRACTS OF JAPAN vol. 2000, no. 26, 1. Juli 2002 (2002-07-01) & JP 2001 245814 A (MATSUSHITA ELECTRIC IND CO LTD), 11. September 2001 (2001-09-11)

## Beschreibung

Die Erfindung betrifft eine Dusch- oder Wannenvorrichtung mit einem Rohrsystem für einen Warmwasserkreislauf, das auch Totwasserbereiche umfasst, in denen eine vollständige Entleerung vor einer erneuten Inbetriebnahme des Wasserkreislaufes nicht gewährleistet ist und Totwasser verbleibt und bei dem die warmwasserführenden Rohre aus Kunststoff gefertigt sind.

Rohrsysteme von Dusch- und Wannenvorrichtungen mit WasserDampf- und Luftkreisläufen, insbesondere so genannter Whirlpool-Bäder, werden nach der Benutzung gesperrt und entleert. Eine vollständige Entleerung ist jedoch nicht erreichbar, da hierzu das Rohrsystem getrocknet werden müßte. Auch erfordert das Funktionsprinzip vorhandener Wasserpumpen, dass diese nicht im Trockenlauf betrieben werden können. Das bedeutet, daß in bestimmten Teilen der Rohrsysteme eine geringe Restwassermenge verbleibt. Die Anwesenheit von Feuchte und Nässe in den Rohrsystemen führt zum Wachstum von Krankheitserregern, insbesondere der gefürchteten Legionella-Bakterien. An Innnenoberflächen der Rohrsysteme bilden sich somit ständig Bakterien und Pilze, die in diesem Milieu existieren und sich ausweiten. Mit erneuter Inbetriebsetzung gelangen damit derartige Bakterien und Pilze in das Badewasser.

In der Praxis finden bei Wannenanlagen Verfahren Anwendung, die das Wachstum der Krankheitserreger verhindern sollen. Die DE 41 08 539 beschreibt ein Verfahren, bei dem ein nebelförmiges , strömungsfähiges Medium erzeugt wird, welches durch das Rohrsystem hindurchgeführt wird. Zur Herstellung des Mediums wird keimfreies Wasser verwendet. Eine solche Bearbeitung ist relativ umständlich und hat darüber hinaus den Nachteil, dass die Rohrsysteme, die nach der Reinigung nicht vollständig entleert werden können, weiterhin den Krankheitserregern in der Zeit bis zur nächsten Benutzung einen Nährboden zu bieten.

Das US-Patent 5,919,554 beschreibt allgemein antibakterielle, mit Glasfasern verstärkte Polyester-Kunststoffverbunde (FRP) und speziell FRP-Verbunde mit antibakteriellen Bestandteilen oder Chemikalien, die im Polyesterharz eingebettet sind. Die Verbunde werden durch diese Maßnahme mit einem lebenslangen antibakteriellen Schutz versehen. Aus diesem Werkstoff werden Produkte gefertigt, die ständig mit Wasser oder anderen Flüssigkeiten im Kontakt stehen. Hierzu gehören z. B. Wannen, Waschbecken und Bodenabläufe in Bädern, aber auch Boote und Swimmingpools. Das chemische Gleichgewicht ist die Grundlage, dass das in den amorphen Zonen integrierte antibakterielle Additiv an die Oberfläche des Bauteils wandert (diffundiert) und dort gegen die Krankheitserreger wirkt.

Der Nachteil dieser Erfindung ist das aufwendige Herstellungsverfahren, welches für Massenartikel wie Rohre nicht geeignet ist. Andererseits ist bedenklich, ob ständig dem Badewasser eine antibakterielle Substanz zuzufügen ist, da hiermit leicht allergische Reaktionen bei den badenden Personen hervorgerufen werden können.

Auch der GB 2 353 283 A sind antibakterielle Kunststoffe zu entnehmen, die mit Kochsalz als hauptsächlichem antibakteriellen Wirkstoff versetzt sind. Es werden zahlreiche Anwendungen genannt, auch die Anwendung bei Rohren, ohne jedoch auf die Problematik bei Totwasserbereichen einzugehen.

Eine Kombination aus Arsen-Verbindung mit Vitamin E als antibakteriellem Zusatz zu Kunststoffen schlagen die Erfinder der US 5 554 373 A vor. Hier wird schon darauf hingewiesen, dass bei stehenden Wässern in städtischen Wasserleitungen sich Bakterien an den Rohrleitungen vermehren können und mit dem Frischwasser dann ausgeschwemmt werden. Spezielle Lösungen für Dusch- oder Wannenvorrichtungen werden nicht genannt. Es wird vorausgesetzt, dass jeweils das gesamte, in Frage kommende Rohrsystem mit derartigen antibakteriell ausgerüsteten Rohren verlegt wird.

Aus der EP0875230 ist eine Whirlpool-Vorrichtung bekannt, die eine Rezirkulationspumpe, ein Ansaugrohr sowie Wasserstrahl- und Luftdüsen aufweist. Um die Ansiedlung von Bakterien, die aufgrund des verwendeten warmen Wassers in dem Rohrsystem eines Whirlpools oder allgemein einer Dusch- oder Wannenvorrichtung gute Lebensbedingungen vorfinden, zu verhindern, schlägt die EP0875230 vor, die Vorrichtung so auszugestalten, dass diese keine Totwasserbereiche aufweist, sondern vollständig entleert werden kann. Zusätzlich, so schlägt die EP0875230 vor, können alle Oberflächen der Whirlpool-Vorrichtung, die mit Luft oder Wasser in Berührung kommen, aus Plastikmaterialien hergestellt sein, die einen anti-bakteriellen Wirkstoff enthalten.

Die vollständige Entleerung ist jedoch, wie eingangs geschildert, kaum oder, wenn überhaupt, nur mit hohem technischen Aufwand zu erzielen. Die anti-bakterielle Ausrüstung aller Oberflächen wiederum führt dazu, dass dem Badewasser ständig antibakterielle Substanzen zugefügt werden, was allergische Reaktionen bei den badenden Personen auslösen kann.

Der Erfindung liegt die Aufgabe zugrunde, Krankheitserregern in nicht vollständig entleerten Rohrsystemen bei Dusch- oder Wannenvorrichtungen mit Wasser- und Luftkreisläufen abzutöten wobei nur eine relativ geringe Menge an Wasser mit antibakterieller Substanz versetzt wird.

Erfindungsgemäß wird die Aufgabe durch die Merkmale des Anspruchs 1 gelöst.

Demgemäß ist eine Dusch- oder Wannenvorrichtung vorgesehen, die ein Rohrsystem für einen Warmwasserkreislauf aufweist, das auch Totwasserbereiche umfasst, in denen eine vollständige Entleerung vor einer erneuten Inbetriebnahme des Wasserkreislaufes nicht gewährleistet ist und Totwasser verbleibt, und bei dem die warmwasserführenden Rohre aus Kunststoff gefertigt sind. Zumindest ein Teil der warmwasserführenden Rohre ist aus einem Kunststoff gefertigt, welchem vor oder bei der Herstellung ein anti-bakterieller Wirkstoff beigefügt ist. Die Vorrichtung ist dadurch gekennzeichnet, dass ausschließlich in den Totwasserbereichen des Rohrsystems Rohre aus besagtem mit einem antibakteriellen Wirkstoff versetzten Kunststoff verwendet werden, und dass Kunststoff, anti-bakterieller Wirkstoff und Herstellungsverfahren so gewählt sind, dass der Wirkstoff bei Temperaturen oberhalb der Zimmertemperatur aus dem Kunststoff in das Totwasser diffundiert.

Die Rohre sind vorzugsweise aus einem thermoplastischen Kunststoff, insbesondere aus Polyvinylchlorid (PVC), extrudiert, welchem vor der Extrusion ein antibakterieller Wirkstoff beigefügt wird. PVC wird auch bei konventionellen Rohren bevorzugt. Die Werkstoffe der konventionellen Rohre und der antibakteriellen Rohre sollten gleiche Eigenschaften besitzen, damit eine Kombination ohne Wechselwirkungen problemlos möglich ist.

Den Rohren wird an ihrer Innenseite mittels eines eine aufgerauhte Struktur im Bereich der Oberfläche erzeugenden Verfahrens eine Übergangsflächenvergrößerung vermittelt, damit die antibakteriell wirkende Oberfläche vergrößert wird. Die Übergangsflächenvergrößerung wird vorzugsweise mittels einer Corona-Behandlung erzeugt.

Der Kunststoff enthält vorzugsweise eine Menge von 0,1 bis 5 Gew.-% eines antibakteriellen Stoffes. Bei diesen Mengen ist erfahrungsgemäß die antibakterielle Wirkung ausreichend vorhanden, ohne dabei andere physikalische und physiologische Eigenschaften zu verschlechtern.

Als antibakterieller Wirkstoff wird vorzugsweise 5-Chlor-2-(2,4-dichlorophenoxy)phenol gewählt, da die flüssige Phase bei ca. 57°C bis 74°C und der Verdampfungspunkt bei 204°C liegt und die Zugabe während der Herstellung des Rohrwerkstoffs bzw. der Extrusion der Rohre möglich ist, weil die Temperatur zur Kunststoffherstellung bzw. Umformung normalerweise bei ungefähr 170°C liegt.

Die Erfindung wird nachstehend anhand einer Zeichnung, die ein Ausführungsbeispiel wiedergibt, näher erläutert. Es zeigen:
Figuren 1a - 1b typische Totwasserbereiche eines Leitungssystems;
Fig.2 einen schematisierten Querschnitt durch ein Leitungssystem.

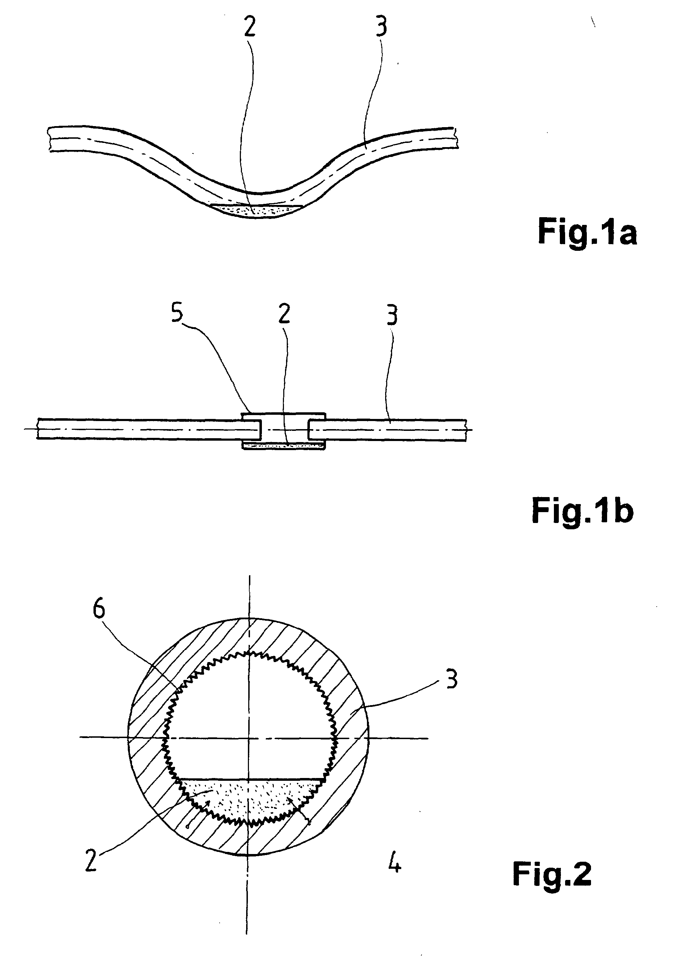

Fig.1a zeigt den Totwasserbereich 2 einer durchhängenden entleerten Rohrleitung 3 und Fig.1b stellt den Totwasserbereich 2 einer Doppelmuffe 5 in einer entleerten Rohrleitung dar. Im schematischen Querschnitt (Fig.2) einer Rohrleitungen 3 weisen die Innenseiten der Rohrleitung eine Übergangsflächenvergrößerung 6 durch Herstellung einer aufgerauhten Innenfläche auf.

Ein antibakterieller Wirkstoff 4, hier 5-Chlor-2-(2,4-dichlorophenoxy)phenol, diffundiert aus der Rohrleitung 3 aus Kunststoff an die Oberfläche der Innenseite und wirkt antibakteriell auf den Totwasserbereich 2.

## Patentansprüche

1. Dusch- oder Wannenvorrichtung mit einem Rohrsystem (1) für einen Warmwasserkreislauf, das auch
Totwasserbereiche (1) umfasst, in denen eine vollständige Entleerung vor einer erneuten Inbetriebnahme des Wasserkreislaufes nicht gewährleistet ist und Totwasser verbleibt und bei dem die warmwasserführenden Rohre (3) aus Kunststoff (5) gefertigt sind,
wobei zumindest ein Teil der warmwasserführenden Rohre aus einem Kunststoff gefertigt ist, welchem vor oder bei der Herstellung ein anti-bakterieller Wirkstoff (4) beigefügt ist,
**dadurch gekennzeichnet,**
**dass** ausschließlich in den Totwasserbereichen (1) des Rohrsystems Rohre aus besagtem mit einem antibakteriellen Wirkstoff versetzten Kunststoff (4) verwendet werden, und
**dass** der anti-bakterielle Wirkstoff (4) bei Temperaturen oberhalb der Zimmertemperatur aus dem Kunststoff in das Totwasser (2) diffundiert.

2. Dusch- oder Wannenvorrichtung mit einem Rohrsystem nach Anspruch 1, **dadurch gekennzeichnet, dass** die Rohre aus Polyvinylchlorid (PVC) extrudiert sind, welchem vor der Extrusion der antibakterielle Wirkstoff (4) beigefügt wird.

3. Dusch- oder Wannenvorrichtung mit einem Rohrsystem nach Anspruch 1 oder 2, **dadurch gekennzeichnet, daß** der Kunststoff (5) eine Menge von 0,1 bis 5 Gew.-% des antibakteriellen Stoffes (4) enthält.

4. Dusch- oder Wannenvorrichtung mit einem Rohrsystem nach Anspruch 1 bis 3, **dadurch gekennzeichnet, dass** als antibakterieller Wirkstoff (4) 5-Chlor-2-(2,4-dichlorophenoxy)phenol gewählt ist.

5. Dusch- oder Wannenvorrichtung mit einem Rohrsystem nach einem der vorhergehenden Ansprüche, **dadurch gekennzeichnet, dass** den Rohren an ihrer Innenseite mittels eines eine aufgerauhte Struktur im Bereich der Oberfläche erzeugenden Verfahrens eine Übergangsflächenvergrößerung (6) vermittelt ist.

6. Dusch- oder Wannenvorrichtung mit einem Rohrsystem nach Anspruch 5, **dadurch gekennzeichnet, daß** die Übergangsflächenvergrößerung (6) mittels Corona-Behandlung (7) erzeugt ist.

## Claims

1. Shower or bath installation with a pipework system (1) for a hot water circuit which also includes wake regions (1), in which complete drainage before renewed use of the hot water circuit is not guaranteed and wherein the hot water supply pipes (3) are manufactured from plastic (3),
wherein at least some of the water supply pipes are manufactured from a plastic to which an antibacterial agent (4) is added before or during manufacture,
**characterised in that**
pipes made of said plastic (4) mixed with an antibacterial agent are used solely in the wake regions (1) of the pipework system, and
the antibacterial agent (4) diffuses out of the plastic into the wake (2) at temperatures above room temperature.

2. Shower or bath installation with a pipework system according to claim 1, **characterised in that** the pipes are extruded from polyvinyl chloride (PCV) to which the antibacterial agent (4) is added prior to extrusion.

3. Shower or bath installation with a pipework system according to claim 1 or 2, **characterised in that** the plastic (5) contains 0.1 to 5% by weight of the antibacterial substance (4).

4. Shower or bath installation with a pipework system according to claim 1 or 3, **characterised in that** 5-chlorine-2-(2,4-dichlorophenoxyl)phenol is selected as the antibacterial agent (4).

5. Shower or bath installation with a pipework system according to one of the preceding claims, **characterised in that** contact area enlargement is imparted to the inner side of the pipes (6) by means of a method producing a roughened structure in the region of the surface.

6. Shower or bath installation with a pipework system according to claim 5, **characterised in that** the contact area enlargement (6) is produced by means of corona treatment (7).

## Revendications

1. Dispositif de douche ou de baignoire incluant un système de tuyauterie (1) de circulation d'eau chaude qui comprend également des zones d'eau morte (2), dans lesquelles un vidage total n'est pas garanti avant une nouvelle mise en oeuvre de la circulation d'eau, et des eaux mortes subsistent, et dans lequel les tuyaux (3) d'amenée d'eau chaude sont fabriqués en matière plastique (5),
dans lequel au moins une partie des tuyaux d'amenée d'eau chaude est fabriquée en une matière plastique à laquelle un agent actif antibactérien (4) est ajouté avant ou pendant la fabrication,
**caractérisé en ce que**
ce sont exclusivement des tuyaux constitués de la matière plastique (4) mélangée avec un agent actif antibactérien mentionnée ci-dessus qui sont utilisés dans les zones (2) d'eaux mortes du système de tuyauterie, et **en ce que**
l'agent actif antibactérien (4) se diffuse depuis la matière plastique dans l'eau morte (2) à des températures supérieures à la température ambiante.

2. Dispositif de douche ou de baignoire à système de tuyauterie selon la revendication 1, **caractérisé en ce que** les tuyaux sont extrudés à partir d'un chlorure de polyvinyle (PVC) auquel l'agent actif antibactérien (4) est ajouté avant l'extrusion.

3. Dispositif de douche ou de baignoire à système de tuyauterie selon la revendication 1 ou 2, **caractérisé en ce que** la matière plastique (5) contient une teneur de 0,1 à 5% en poids de l'agent antibactérien (4).

4. Dispositif de douche ou de baignoire à système de tuyauterie selon l'une quelconque des revendications précédentes, **caractérisé en ce que** l'agent actif antibactérien (4) choisi est le 5-chlore-2(2-4 de dichlorophénoxy)phénol.

5. Dispositif de douche ou de baignoire à système de tuyauterie selon l'une quelconque des revendications précédentes, **caractérisé en ce qu'**un accroissement (6) de la surface de transmission est communiqué aux tuyaux sur leur côté interne au moyen d'un procédé engendrant une structure rendue rugueuse dans la zone de la surface.

6. Dispositif de douche ou de baignoire à système de tuyauterie selon la revendication 5, **caractérisé en ce que** l'accroissement (6) de surface de transmission est engendré par un traitement corona (7).
